# EUROPEAN PATENT SPECIFICATION

(11) **EP 2 349 304 B1**
(45) Date of publication and mention of the grant of the patent: **26.06.2013**
(21) Application number: 09823871.0
(22) Date of filing: 07.08.2009
(51) Int. Cl.: A61K 36/896, A61P 3/10, A61P 9/10

(54) **USES OF CURCULIGO LATIFOLIA (C. LATIFOLIA) EXTRACTS**
VERWENDUNGEN VON EXTRAKTEN AUS CURCULIGO LATIFOLIA (C. LATIFOLIA)
UTILISATIONS D'EXTRAITS DE CURCULIGO LATIFOLIA (C. LATIFOLIA)

(30) Priority: 28.10.2008 MY 0804284
(43) Date of publication of application: 03.08.2011
(73) Proprietor: Universiti Putra Malaysia, 43400 Selangor Darul Ehsan (MY)
(72) Inventor: ISMAIL, Maznah, 43400 Selangor Darul Ehsan (MY); ISHAK, Nur, Akmal, 43400 Selangor Darul Ehsan (MY); HAMID, Muhajir, 43400 Selangor Darul Ehsan (MY)
(74) Representative: Dilg, Haeusler, Schindelmann Patentanwaltsgesellschaft mbH
(86) International application number: PCT/MY2009/000112
(87) International publication number: WO 2010/050793

(56) References cited:
- US-A- 5 178 899
- US-A- 5 395 921
- US-A1- 2008 107 787
- MADHAVAN V ET AL: "Antidiabetic activity of Curculigo orchioides root tuber", PHARMACEUTICAL BIOLOGY, vol. 45, no. 1, January 2007 (2007-01), pages 18-21, XP009158821, ISSN: 1388-0209
- KANT RAVI: 'Sweet proteins-potential replacement for artificial low calorie sweeteners' NUTRITIONAL JOURNAL vol. 4, no. 5, 2005, pages 1 - 6, XP021007678
- MASUDA TETSUYA ET AL.: 'Developments in biotechnological production of sweet proteins' JOURNAL OF BIOSCIENCE AND BIOENGEERING. vol. 102, no. 5, 2006, pages 375 - 389, XP025182978

## Description

### FIELD OF INVENTION

The present invention relates to the uses of *Curculigo latifolia (C. latifolia)* extracts, in the treatment and prevention of metabolic disorder diseases.

### BACKGROUND OF INVENTION

Heterogeneous metabolic disorder such as Type 2 diabetes mellitus is mainly caused by inadequacy of insulin-producing β-cells to keep up with demand and insulin resistance occurring in peripheral tissues such as liver, adipose and muscle tissues. Apart from that, the combinations of sedentary lifestyle, unhealthy dietary habits, and genetic predisposition are the main reasons for this problem. If the condition is not controlled, it can lead to other health problems like obesity, cardiovascular diseases, kidney failures and lost of vision.

Recently, hypoadiponectin, insulin resistance and defect in glucose uptake activity become a central issue in the pathogenesis of type 2. Adiponectin is a cytokine which has been secreted exclusively by adipose tissue during adipocyte differentiation. It plays a role in metabolism to improved insulin sensitivity, glucose tolerance and lipid profile. However, in diabetes situation, the adiponectin level is decrease and it can contribute into insulin resistance and hyperinsulinemia occurrence. Insulin resistance can be defined as insulin-mediated glucose clearance into target tissues which is a peripheral tissue. In normal scenario, insulin induce glucose uptake in these tissues by binding to the insulin receptor proteins at the surface of the cell. Then, it will activate a series of protein within the cell to translocate GLUT4 onto the cell surface to uptake the glucose from the bloodstream into the cells but in type 2 diabetic condition, the glucose uptake activity is being defect and glucose remains in the bloodstream. These mechanisms highlight that hypoadinopectin, hyperinsulinemia and hyperglucosemia are type 2 diabetes symptoms.

Ethnobotanicals have been used widely for many years in East Asian countries mainly in developing countries. Meanwhile, in Western countries, the herbal supplements usage has steadily increased over the last decades. However, there are limited scientific evidences are reported for the efficacy of these ethnobotanicals. Recently, the use of ethnobotanicals in diabetes mellitus treatment has vigorously increased. This emerging interest has led to collaborative works among the scientists to search and study on plants which may have potentials as antidiabetic agents. Many studies have shown that some of ethnobotanicals may improve blood glucose levels in type-2 diabetes mellitus patients and also prevent diabetes-related long term complication. Further studies demonstrated that antidiabetic agents in these plant components display characteristics of insulin sensitization in peripheral tissues and/or insulinotropic action from pancreatic β-cells.

Madhavan and coworkers (Madhavan, V. et al. (2007) Pharmceut. Biol. 45, 18-21) reported that alcoholic and aqueous extracts of the root tuber of *Curculigo orchioides* caused a significant hypoglycemic effect in diabetic rats resulting in a pronounced reduction of blood glucose levels.

*C. latifolia* is a stemless herb that grows in Western Malaysia and its fruits have been used by the natives as sweetener. Interestingly, the sweet taste is from the isolated protein, curculin presence in the *C***.** *latifolia* fruit. This protein exhibits both sweet-tasting and taste-modifying activities. However, there are no proper studies about their potential use neither as an antidiabetic agent nor as an artificial sweetener for diabetic patients since it has been shown that the fruits of *C*. *latifolia* are 9000 times sweeter than sucrose.

Currently, some work showed that artificial sweetener consumptions like saccharine, aspartame and cyclamate have side effects such as psychological problems, mental disorders, bladder cancer, heart failure and brain tumors. In order to overcome this problem, the search for non-carbohydrate sweeteners from natural sources has led to the discovery of many intensely sweet-tasting over artificial sweeteners. Since they are natural, they could be safer than aspartame and other sweeteners. This is a new approach to the potential treatment of diabetes, obesity and other metabolic disorders.

### SUMMARY OF INVENTION

Accordingly, the present invention provides *Curculigo latifolia (C. latifolia)* extracts, wherein the extracts extracted from different parts of a plant such as fruits, roots and leaves, individually and in any combinations thereof, characterized in that the extracts are used as an agent for treating and preventing metabolic disorder diseases such as diabetes mellitus, obesity, cardiovascular, and atherosclerosis.

The present invention consists of several novel features and a combination of parts hereinafter fully described and illustrated in the accompanying description and drawing, it being understood that various changes in the details may be made without departing from the scope of the invention or sacrificing any of the advantages of the present invention.

### BRIEF DESCRIPTION OF THE DRAWINGS

The present invention will be fully understood from the detailed description given herein below and the accompanying drawings which are given by way of illustration only, and thus are not limitative of the present invention, wherein:
Figure 1a shows the effect of *C**.** latifolia* extracts on BRIN-BD11 pancretic cell lines viability;
Figure 1b shows the effect of *C**.** latifolia* extracts on 3T3-L1 adipocyte cell lines viability;
Figure 1c shows the effect of *C**.** latifolia* extracts on L6 myotubes cell lines viability;
Figure 2 shows the effect of *C**.** latifolia* extract on BRIN-BD11 cell in insulin secretion after 30 minutes treatment;
Figure 3a shows the ***C.** latifolia* fruit extract effect on glucose uptake activity in differentiated 3T3-L1 adipocytes in presence and absence of insulin;
Figure 3b shows the ***C.** latifolia* root extract effect on glucose uptake activity in differentiated 3T3-L1 adipocytes in presence and absence of insulin;
Figure 3c shows the ***C.** latifolia* leaf (hot water) extract effect on glucose uptake activity in differentiated 3T3-L1 adipocytes in presence and absence of insulin;
Figure 3d shows the ***C.** latifolia* leaf extract effect on glucose uptake activity in differentiated 3T3-L1 adipocytes in presence and absence of insulin;
Figure 4a shows the ***C.** latifolia* fruit extract effect on glucose uptake activity in differentiated L6 myotubes in presence and absence of insulin;
Figure 4b shows the ***C.** latifolia* root extract effect on glucose uptake activity in differentiated L6 myotubes in presence and absence of insulin;
Figure 4c shows the *C**.** latifolia* leaf (hot water) extract effect on glucose uptake activity in differentiated L6 myotubes in presence and absence of insulin;
Figure 4d shows the *C*. *latifolia* leaf extract effect on glucose uptake activity in differentiated L6 myotubes in presence and absence of insulin;
Figure 5 shows the effect of *C. latifolia* extracts on differentiated 3T3-L1 adipocyte in adiponectin secretion after 30 minutes treatment with absence of insulin.
Figure 6 shows the effect of *C. latifolia* extracts on differentiated 3T3-L1 adipocyte in adiponectin secretion after 30 minutes treatment with presence of insulin.
Figure 7a shows the effect of *C**.** latifolia* extracts on body weight in high fat-fed diet and low dose STZ induced diabetic rats;
Figure 7b shows the effect of *C**.** latifolia* extracts on fasting blood glucose in high fat-fed diet induced diabetic rats;
Figure 7c shows the effect of *C**.** latifolia* extracts on insulin level in high fat-fed diet induced diabetic rats; and
Figure 7d shows the effect of *C**.** latifolia* extracts on adiponectin level in high fat-fed diet induced diabetic rats.

### DETAILED DESCRIPTION OF THE PREFERRED EMBODIMENTS

The present invention relates to the uses of *Curculigo latifolia (C. latifolia)* extracts. Hereinafter, this specification will describe the present invention according to the preferred embodiments of the present invention. However, it is to be understood that limiting the description to the preferred embodiments of the invention is merely to facilitate discussion of the present invention and it is envisioned that those skilled in the art may devise various modifications and equivalents without departing from the scope of the appended claims.

More particularly, the present invention relates to a method for ameliorating blood glucose, glucose uptake activity, insulin and adiponectin level. The treatment involves administration of *C*. *latifolia* extracts.

Accordingly, it is an object of the present invention to provide *C. latifolia* extracts which can modulate diabetes mellitus by administrating an effective amount of *C. latifolia* extracts to the subject, wherein the amount of *C. latifolia* extracts modulates the symptoms of diabetes mellitus.

In order to achieve the above object, the extensive studies in *in vitro* and *in vivo* have been conducted. In *in vitro,* an effective amount of *C. latifolia* extracts have been tested in cell lines: BRIN-BD11, 3T3-L1 adipocytes and L6 myotubes. Meanwhile, in *in vivo, C. latifolia* extracts has been administered orally to subjects.

Accordingly, it is derscribed herein a method of cytotoxicity of BRIN-BD11, 3T3-L1 adipocyte and L6 myotubes treated with *C. latifolia* extracts, wherein the amount of C. *latifolia* extracts is found to be non-toxic towards those cell lines.

Described is also a method of glucose uptake in 3T3-L1 adipocyte and L6 myotubes cell lines treated with *C. latifolia* extracts, wherein the amount of *C. latifolia* extracts is found to increase glucose uptake activity in 3T3-L1 adipocyte and L6 myotubes cell lines.

Furthermore, it is described a method of secretion of insulin in BRIN BD11 cell line and adiponectin in 3T3-L1 adipocytes cell line treated with C. *latifolia* extracts, wherein the amount of C. *latifolia* extracts is found to increase secretion of insulin in BRIN BD11 cell line and adiponectin in 3T3-L1 adipocyte.

According to above objects, the subject has been induced to develop diabetes mellitus by combination of high fat diet (HFD) and low dose streptozotocin (STZ).

Diabetes mellitus is non-insulin dependent diabetes mellitus (NIDDM). The symptoms of diabetes mellitus can be selected from the group that showing hyperglycemia, obesity, increased insulin level, polyphagia, polydipsia and polyuria.

Yet further, subject has been administered orally with an effective dose is about 100 mg/kg body weight per day. More specifically, subject has been administered for a 30 days.

An embodiment of the present invention is reducing blood glucose in a diabetes mellitus subject by administering an effective amount of *C. latifolia* extracts.

According to the method for ameliorating blood glucose level described in the present invention , fasting blood glucose and oral glucose tolerance are monitored.

It is envisioned that the *C. latifolia* extracts reduce blood glucose level and increase insulin and adiponectin levels in the subject.

Further envisioned of the present study, *C. latifolia* extracts increase oral glucose tolerance in the subject.

The *C. latifolia* extracts according to the present invention can be prepared in accordance with the following process.

### Example 1

### Preparation of plant extract

### C. latifolia fruit extract

Fresh *C. latifolia* fruits were washed with distilled water. Then, 50 g of C. *latifolia* fruits were extracted with 2 l of distilled water in 5 I beaker for 24 hours with continuous stirring at room temperature. This extract was filtered through Whatman no.1 filter paper. The filtrate was then collected and lyophilized. The lyophilized sample was kept at -80°C until use.

### C. latifolia leave extract

### Two Types of Extracts Used:

### (1) Hot water extract

Five grams of *C. latifolia* leave powder were placed in 200 ml boiling (distilled) water. Then it was removed from the heat source and allowed to infuse for 15 min. This suspension was filtered with Whatman no. 1 filter paper. The filtrate was then collected and lyophilized. The lyophilized sample was kept at -80°C until use.

### (2) Normal water extract

Five grams of *C. latifolia* leave powder were extracted with 200 ml of distilled water and were soaked for 24 hours with continuous stirring at room temperature. This extract was filtered through Whatman no.1 filter paper. The filtrate was then collected and lyophilized. The lyophilized sample was kept at -80°C until use.

### C. latifolia root extract

Five grams of *C. latifolia* root powder were extracted with 200 ml of distilled water and were soaked for 24 hours with continuous stirring at room temperature. This extract was filtered through Whatman no.1 filter paper. The filtrate was then collected and lyophilized. The lyophilized sample was kept at -80°C until use.

### Example 2

### In vitro cytotoxicity study of C. latifolia extracts tested on 3T3-L1 adipocyte, L6-myotubes and BRIN-BD11 pancreatic cell lines

### Cell culture

BRIN-BD11 cell line was cultured and maintained in RPMI 1640 medium supplemented with 10% foetal bovine serum, 1% penicillin-streptomycin and 1% glutamine at 37°C in a humidified atmosphere of 5% CO₂. Meanwhile, 3T3 adipocytes and L6 myotubes cells were cultured in Dulbecco's modified Eagle's medium (DMEM) supplemented with streptomycin/penicillin antibiotics and 10% fetal bovine serum. Both cells were maintained in humidified atmosphere of 5% CO₂ at 37°C. Cells were subcultured every 2 to 3 days at approximately 80% confluence using trypsin-EDTA to detach the cell from the culture flask. Cell counting was done using hemocytometer. For differentiation, L6 cells were transferred to DMEM with 2% fetal bovine serum, 4-6 days post-confluence. The extent of differentiation was established by observing multinucleation of cells and >90% fusion of myoblasts into myotubes were considered.. Meanwhile, differentiation of 3T3 preadipocyte was grown in the plates to reach confluence in 3 days. At this point (day 0) cells were switched to differentiation medium (DMEM, 10% FBS, 0.25 µM dexamethasone, 0.25 mM IBMX, and 1 µg/ml insulin) for 3 days, with one medium change in between. On day 3, the dexamethasone and IBMX were removed leaving insulin on the cells for an additional 4 days, changing the medium every 2 days. Thereafter the cells were maintained in the original propagation DMEM, changing medium every 2-3 days, until use. Plates where cells were >90% differentiated were used for experiments between days 9 to 12 post-induction.

### Cell viability assay

To date various methods have been developed and introduced to measure the viability cell. The CellTiter 96® aqueous non-radioactive cell proliferation assay (Promega, Madison, WI) is one of them. The cells were seeded onto 96-well plates at a concentration 2×10⁵ cells/well in 100 µl of medium culture and allowed to attach for 24 hours. The cells monolayer were washed with phosphate-buffered saline (PBS) to remove unattached cells; the attached cells were incubated in fresh serum free media with different concentrations of *C. latifolia* fruit, leave and root extracts for 72 hours. Cells were then incubated with 20 µl of tetrazolium salt solution for four hours. The absorbance of each well was measured at 490 nm using a Microplate reader (Opsys MR, Thermolabsystems) to quantify the formazon product. The number of living cells in culture is proportional to quantity of formazon product presence.

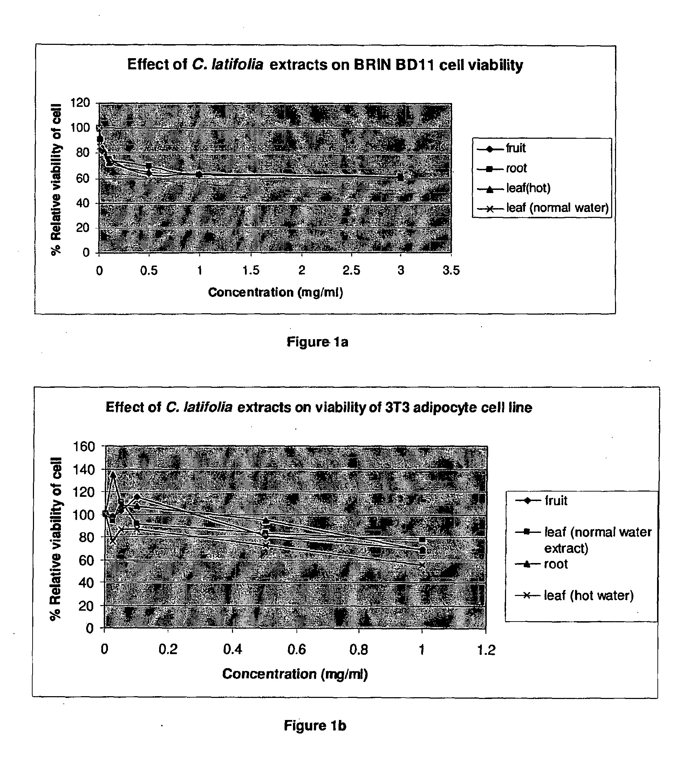

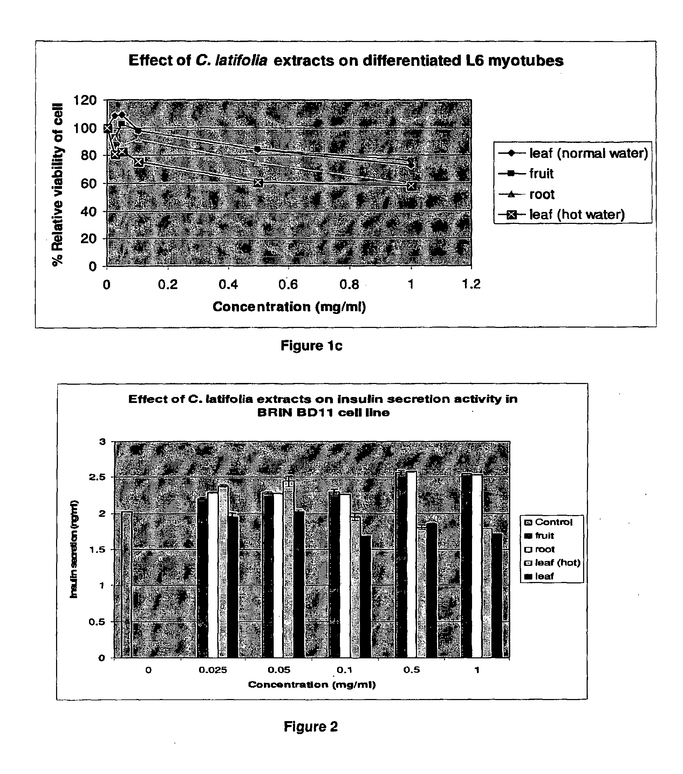

Figures 1a, 1b and 1c show the effect of *C. latifolia* extracts on BRIN BD11, 3T3-L1 adipocyte and L6 myotubes cell lines, respectively.

### Example 3

### Insulin secretion in vitro

BRIN-BD11 cell line was used to evaluate insulin secretion. Insulin secretion activity was determined using 24-well plates. Cells were seeded at a concentration 2×10⁵ cells/well in RPMI 1640 containing 10% foetal bovine serum, 1% antibiotic and 1% glutamine and allowed attachment overnight. Cell were then washed thrice with Kreb's-Ringer bicarbonate buffer (KRB; 115 mM NaCl, 4.7 mM KCI, 1.28 mM CaCl2, 1.2 mM KH₂PO₄, 1.2 mM MgSO₄, 24 mM NaHCO₃, 10 mM Hepes-free acid, 1 g/I bovine serum albumin, 1.1 mM glucose; pH 7.4) and preincubated for 40 minutes in KRB buffer at 37°C. Cells were then incubated for 30 min with 1 ml KRB buffer in the absence and presence of *C. latifolia* extracts and glibenclamide as positive control. Aliquots were removed from each well and stored at -20°C for insulin assay later.

### Insulin assay

In order to quantify the insulin concentration, aliquot from insulin secretion in *in vitro* study were used and assay was done using Mercodia Rat Insulin ELISA (Uppsala, Sweden) protocol. The absorbance of each well was measured at 450 nm using a Microplate reader (Opsys MR, Thermolabsystems) to quantify the insulin concentration.

Figure 2 shows the effect of *C. latifolia* extract on BRIN-BD11 cell in insulin secretion after 30 minutes treatment.

### Example 4

### 2-deoxy-D-[1-³H] glucose (2-DOG) uptake

Glucose uptake was measured in fully differentiated L6 myotubes and 3T3 adipocyte. Cells were rinsed thrice with Krebs-Ringer HEPES buffer (pH 7.4) before treated with *C. latifolia* extracts in the presence and absence of insulin (100 nM). This treatment was allowed to proceed for 30 min. After 30 min, 1 µCi/ml of 2-deoxy-D-[1-³H] glucose was added and allowed 30 min incubated. Before the cells were digested, the medium was collected to vials and frozen at -20°C for adiponectin analysis and the collected process has done on a bed of ice. Then, plates were washed thrice with ice-cold KRH buffer and cells were digested with 0.1% SDS. An aliquot was used to measure the radioactivity by using scintillation counter (Tri-Carb 2300TR, Perkin Elmer Life and Analytical Services, Boston, MA, USA) using Ultima Gold ™ LLT as the scintillation cocktail (Perkin Elmer, Boston, MA, USA). Glucose uptake was expressed as disintegrations per minute (dpm).

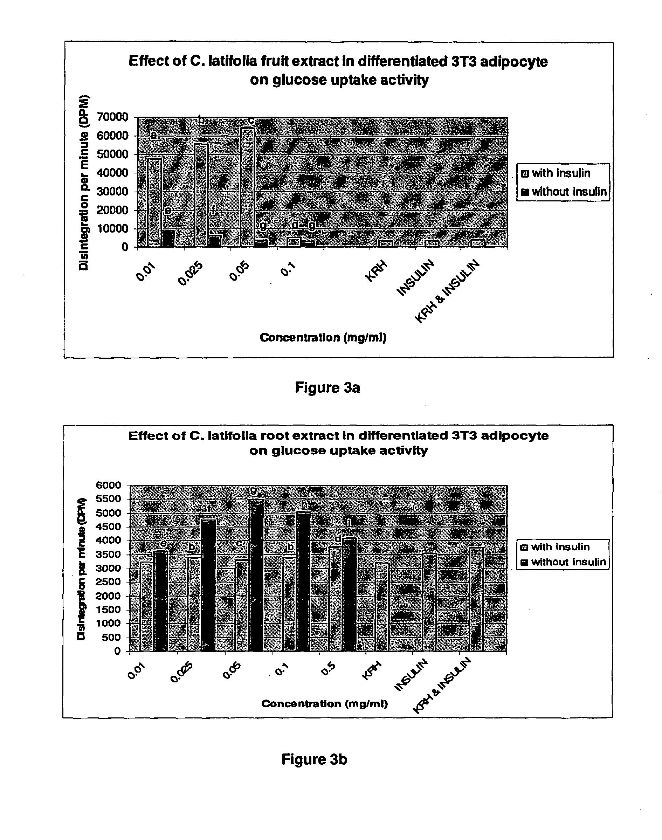

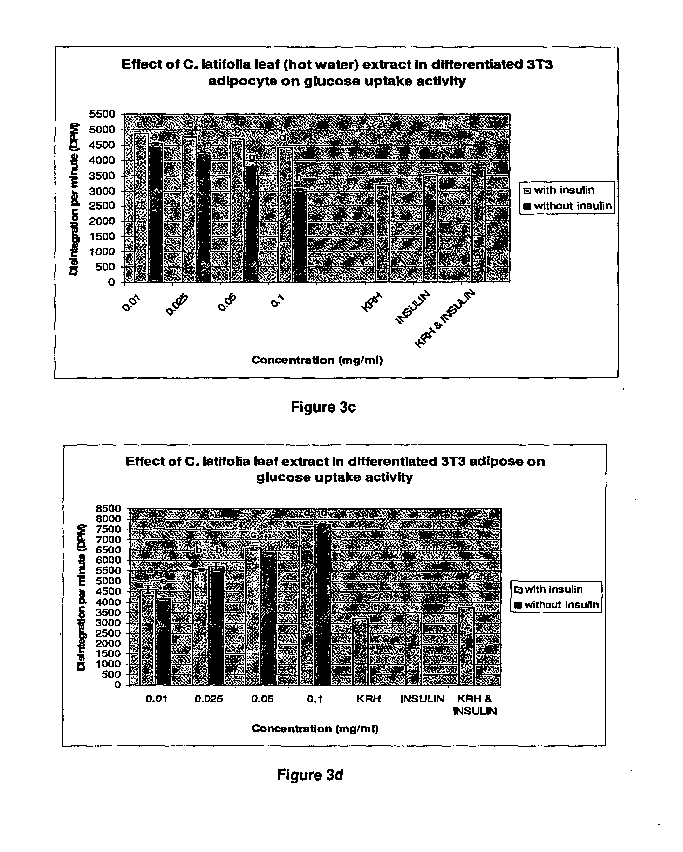

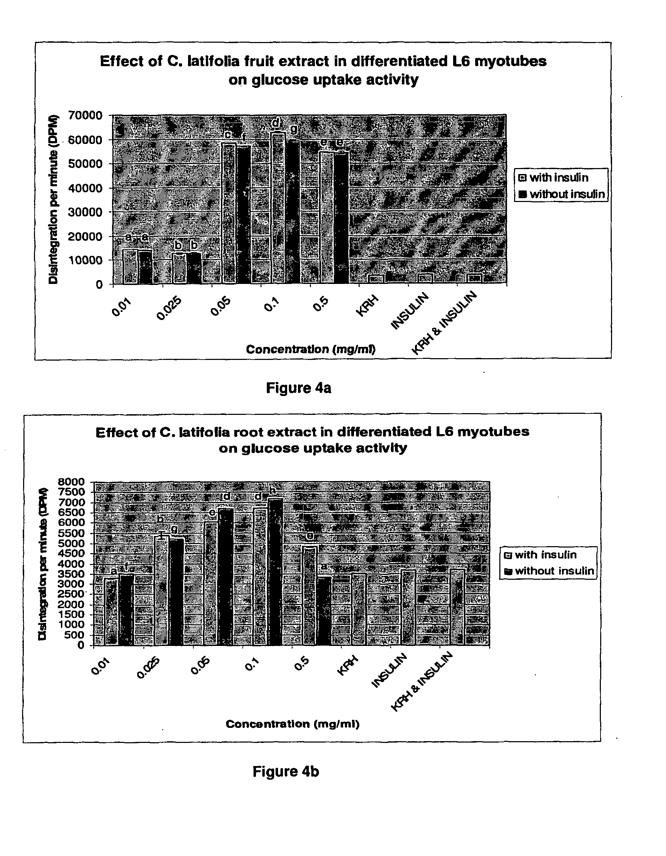

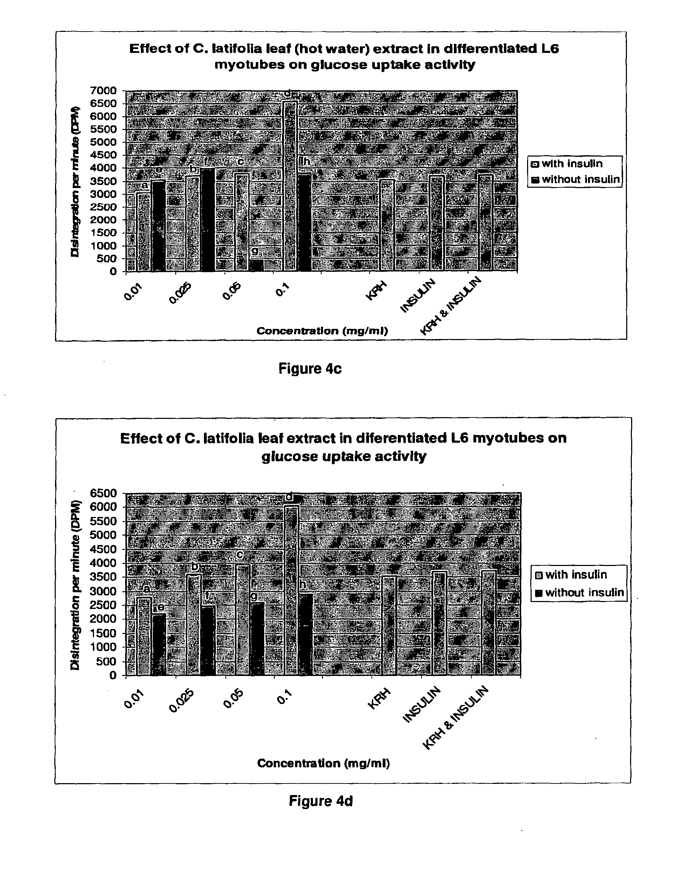

Figures 3a, 3b, 3c and 3d show the *C. latifolia* extracts effect on glucose uptake activity in differentiated 3T3 adipocytes, respectively, whereas, Figures 4a, 4b, 4c and 4d show the *C. latifolia* extracts effect on glucose uptake activity in differentiated L6 myotubes, respectively.

### Example 5

### Adiponectin assay

BioVision Rat Adiponectin ELISA assay (Mountain View, USA) was used to screen C. *latifolia* extracts to potentiate the adiponectin secretion in differentiated 3T3 adipocytes. Aliquot collected from glucose uptake assay were used and assay was done according to the kit procedure.

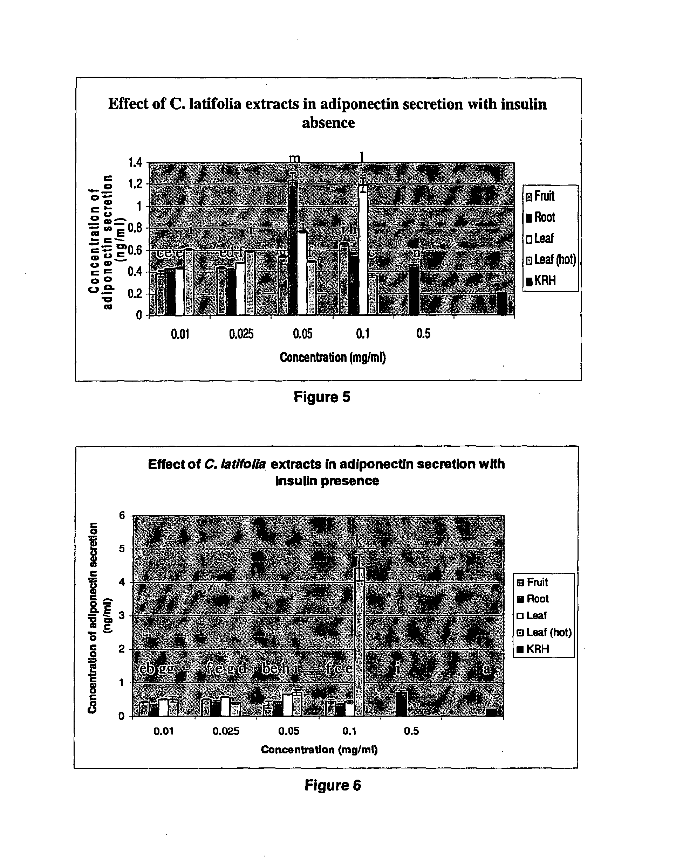

Figure 5 shows the *C. latifolia* extracts effect on adiponectin secretion activity in differentiated 3T3 adipocytes with absence of insulin whereas Figure 6 shows the C. *latifolia* extracts effect on adiponectin secretion activity in differentiated 3T3 adipocytes with presence of insulin.

### Example 6

### Animals

A total of 42 male Sprague-Dawley (SD) rats (160-200g) were used for this study. They were housed individually in polypropylene cages and maintained under controlled room temperature (22±2°C) and humidity (55±5%) with 12:12 h light and dark cycle. All rats were provided with free access to water and commercially available rat normal pallet (NPD) prior to acclimatize period. All experimental protocols for animal care and use was approved by the Animal Care and Use Committee (ACUC) of Faculty of Medicine and Health Sciences, Universiti Putra Malaysia.

### Experimental design

### Phase I

### Preparation of high-fat (HF) diet

The HF diet was formulated based on the composition provided by Levin et. al. (1989). It will be prepared from a mixture of 50% normal rat chow pallet, 24% of corn oil (Mazola brand), 20% of full-cream milk powder (Nespray brand from Nestlé) and 6% sugar.

### Development of HFD-fed and low dose STZ- treated type 2 diabetic rats

Rats were allocated into two groups based on dietary regimens; NPD and HFD and they were fed for a month. After a month on either diet, rats which were introduced with HFD will be anesthetized with diethyl ether after an overnight fast and then inject with STZ (35 mg/kg) via intravenous. Rats were continued to their original diets (chow or fat) and water after the STZ injection. After seven days of STZ injection, diabetes conditions were identified by polydipsia, polyuria and by measuring fasting blood glucose level; glucose level >170 mg/dl were included in the study.

Body weight, food consumption and fasting blood glucose were determined weekly.

### Phase II

The animals are randomly divided into seven groups of three animals each.
- Group 1 : Normal control ( normal pellet diet, non-diabetic, untreated) rats
- Group 2 : Diabetic control ( high fat-fed diet, diabetic, untreated) rats
- Group 3 : Diabetic control (high fat-fed diet, induced with STZ, diabetic, untreated) rats
- Group 4 : Diabetes test rats (high fat-fed diet, induced with STZ, diabetic, treated), treatment with glibenclimide 600µg per b.w
- Group 5 : Diabetic test rats (high fat-fed diet, induced with STZ, diabetic, untreated), treatment with *C. latifolia* fruit extract 100mg per b.w
- Group 6 : Diabetic test rats (high fat-fed diet, induced with STZ, diabetic, untreated), treatment with *C. latifolia* root extract 100mg per b.w
- Group 7 : Diabetic test rats (high fat-fed diet, induced with STZ, diabetic, untreated), treatment with combination of *C. latifolia* root and fruit extract 100mg per b.w

Body weight, food consumption and fasting blood glucose are determined weekly.

### Oral glucose tolerance test (OGTT)

Fasting blood glucose level of each rat was determined at zero-time (after overnight fasting with free access to water). Glucose (2g/kg b.w) was orally administered 30 minutes after an oral administration of the *C. latifolia* extracts or glibenclamide. Blood glucose concentration was measured just before and 30, 60 and 120 minutes after the oral administration of test sample.

### Biological assays

Blood samples were collected at the end of every phase of treatments. Plasma was collected by cardiac puncture for various biological assays. Physical and biochemical parameters were obtained such as changes in body weights, food intake, plasma glucose, insulin and adiponectin level. Insulin level was measured using an Insulin ELISA kit (Mercodia AB, Uppsala, Sweeden) with rat insulin as a standard. Adiponectin level is measured using BioVision Rat Adiponectin ELISA assay (Mountain View, USA).

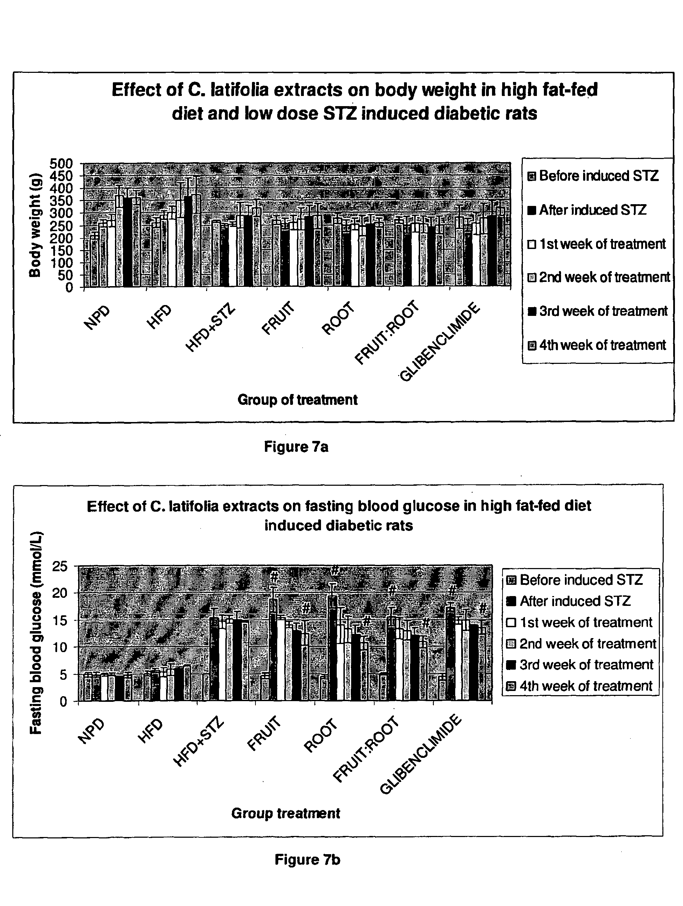

Figure 7a shows the effect of *C. latifolia* extracts on body weight in high fat-fed diet and low dose STZ induced diabetic rats whereas Figure 7b shows the effect of *C. latifolia* extracts on fasting blood glucose in high fat-fed diet induced diabetic rats.

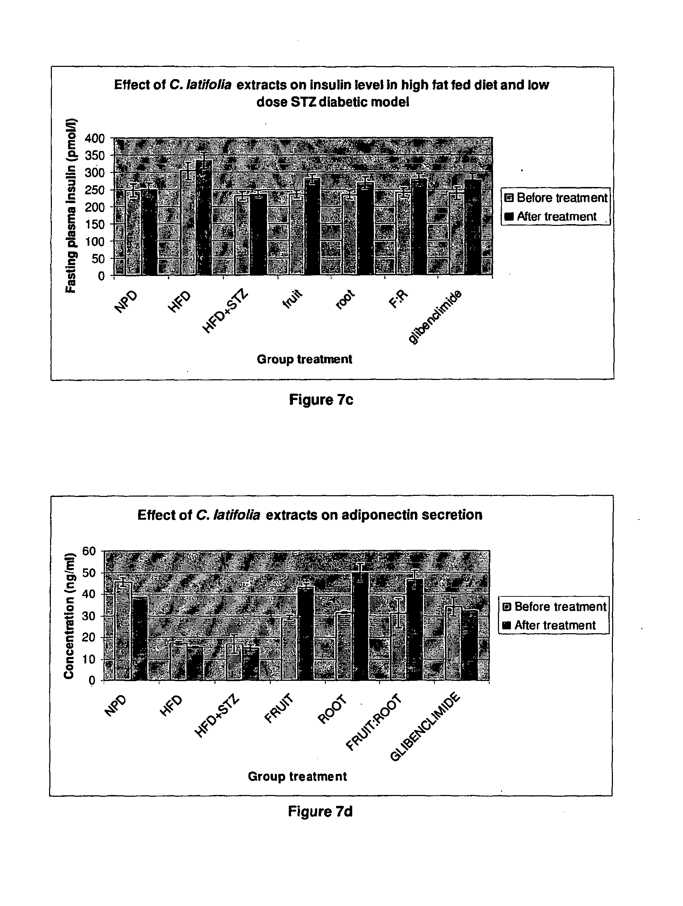

On the other hand, Figure 7c shows the effect of *C. latifolia* extracts on insulin level in high fat-fed diet induced diabetic rats whereas Figure 7d shows the effect of *C. latifolia* extracts on adiponectin level in high fat-fed diet induced diabetic rats

In specific embodiments, the present invention relates to:
Embodiment 1: *Curculigo latifolia (C. latifolia)* extracts, wherein the extracts extracted from different parts of a plant such as fruits, roots and leaves, individually and in any combinations thereof, characterized in that the extracts are used as an agent for treating and preventing metabolic disorder diseases such as diabetes mellitus, obesity, cardiovascular, and atherosclerosis.
Embodiment 2: *C. latifolia* extracts according to embodiment 1, wherein fresh and dried fruits, roots and leaves are extracted with distilled water.
Embodiment 3: A use of an effective amount of *C. latifolia* extracts in the manufacture of a medicament for treating and preventing metabolic disorder diseases such as diabetes mellitus, obesity, cardiovascular, and atherosclerosis in a subject in need thereof.
Embodiment 4: A use according to embodiment 3, wherein the subject is suffering from diabetes mellitus.
Embodiment 5: A use according to embodiment 4, wherein the diabetes mellitus is Type I or Type II diabetes
Embodiment 6: A use according to any one of dembodiments 2 to 6 wherein the extracts are administered orally or intravenously.

## Claims

1. *Curculigo latifolia (C. latifolia)* extracts for use in the treatment and prevention of metabolic disorder diseases, wherein the extracts are extracted from different parts of a plant, individually and in any combinations.

2. The *C. latifolia* extracts for use according to claim 1, wherein the different parts of a plant are selected from the group consisting of fruits, roots, and leaves.

3. The *C. latifolia* extracts for use according to claim 2, wherein fresh and dried fruits, roots and leaves are extracted with distilled water.

4. The *C. latifolia* extracts for use according to any one of claims 1 to 3, wherein the metabolic disorder diseases are selected from the group consisting of diabetes mellitus, obesity, cardiovascular diseases, and atherosclerosis.

5. The *C. latifolia* extracts for use according to claim 4, wherein the metabolic disorder disease is diabetes mellitus.

6. The *C. latifolia* extracts for use according to claim 5, wherein the diabetes mellitus is Type I or Type II diabetes

7. The *C. latifolia* extracts for use according to any one of claims 1 to 6 wherein the extracts are administered orally or intravenously.

8. The *C. latifolia* extracts for use according to any one of claims 1 to 7, wherein the treatment or prevention involves any one of ameliorating blood glucose, glucose uptake activity, insulin level, and adiponectin level.

## Patentansprüche

1. *Curculigo latifolia (C. latifolia)* Extrakte für eine Verwendung in der Behandlung und der Prävention von metabolischen Funktionsstörungserkrankungen, wobei die Extrakte aus unterschiedlichen Teilen einer Pflanze extrahiert sind, individuell und in jeglichen Kombinationen.

2. Die *C. latifolia* Extrakte für eine Verwendung gemäß Anspruch 1, wobei die unterschiedlichen Teile einer Pflanze aus der Gruppe bestehend aus Früchten, Wurzeln und Blättern ausgewählt sind.

3. Die *C. latifolia* Extrakte für eine Verwendung gemäß Anspruch 2, wobei frische und getrocknete Früchte, Wurzeln und Blätter mit destilliertem Wasser extrahiert werden.

4. Die *C. latifolia* Extrakte für eine Verwendung gemäß irgendeinem der Ansprüche 1 bis 3, wobei die metabolischen Funktionsstörungserkrankungen aus der Gruppe bestehend aus Diabetes mellitus, Adipositas, kardiovaskuläre Erkrankungen und Arteriosklerose ausgewählt sind.

5. Die *C. latifolia* Extrakte für eine Verwendung gemäß Anspruch 4, wobei die metabolische Funktionsstörungserkrankung Diabetes mellitus ist.

6. Die *C. latifolia* Extrakte für eine Verwendung gemäß Anspruch 5, wobei der Diabetes mellitus Typ I oder Typ II Diabetes ist.

7. Die *C. latifolia* Extrakte für eine Verwendung gemäß irgendeinem der Ansprüche 1 bis 6, wobei die Extrakte oral oder intravenös verabreicht werden.

8. Die *C. latifolia* Extrakte für eine Verwendung gemäß irgendeinem der Ansprüche 1 bis 7, wobei die Behandlung oder die Prävention irgendeines von Ameliorieren des Blutzuckers, der Glukoseaufnahmeaktivität, des Insulinspiegels und des Adiponektinspiegels involviert.

## Revendications

1. Utilisation d'extraits de *Curculigo latifolia (C. latifolia)* dans le traitement et la prévention des maladies de troubles métaboliques, dans laquelle les extraits sont extraits des différentes parties d'une plante, individuellement et dans toutes les combinaisons.

2. Utilisation d'extraits de *C. latifolia* selon la revendication 1, dans laquelle les différentes parties d'une plante sont choisies dans le groupe composé de fruits, racines et feuilles.

3. Utilisation d'extraits de *C. latifolia* selon la revendication 2, dans laquelle sont extraits des fruits frais et secs, racines et feuilles avec de l'eau distillée.

4. Utilisation d'extraits de *C. latifolia* selon l'une quelconque des revendications 1 à 3, dans laquelle les maladies de troubles métaboliques sont choisies dans le groupe composé de diabète mellitus, obésité, maladies cardiovasculaires et d'athérosclérose.

5. Utilisation d'extraits de *C. latifolia* selon la revendication 4, dans laquelle la maladie de trouble métabolique est le diabète mellitus.

6. Utilisation d'extraits de *C. latifolia* selon la revendication 5, dans laquelle le diabète mellitus est du Type I ou le diabète du Type II.

7. Utilisation d'extraits de *C. latifolia* selon l'une quelconque des revendications 1 à 6, dans laquelle les extraits sont administrés par voie orale ou intraveineuse.

8. Utilisation d'extraits de *C. latifolia* selon l'une quelconque des revendications 1 à 7, dans laquelle le traitement ou la prévention est appliqué à n'importe lequel de l'amélioration de la glycémie, de l'activité d'absorption de glucose, de l'insuline et de l'adiponectine.
